# EUROPEAN PATENT APPLICATION

(11) **EP 0 684 565 A1**
(43) Date of publication of application: **29.11.1995**
(21) Application number: 95107281.8
(22) Date of filing: 13.05.1995
(51) Int. Cl.: G06F 17/30, G11B 27/026

(54) **Medical image archiving of lossy and lossless images on a recordable CD**

(30) Priority: 27.05.1994 US 250089
(71) Applicant: EASTMAN KODAK COMPANY, Rochester, New York 14650 (US)
(72) Inventor: Dhurjaty, Sreeram, c/o Eastman Kodak Co., Rochester, New York 14650-2201 (US); Condit, Paul Brainard, c/o Eastman Kodak Co., Rochester, New York 14650-2201 (US); Pelanek, Geraldine Ann, c/o Eastman Kodak Co., Rochester, New York 14650-2201 (US)
(74) Representative: Schmidt, Peter, Dipl.-Ing.

(57) **Abstract**

A transportable medical image storage medium includes a recordable optical compact disk (10). The compact disk (10) has a first storage area (18) which records a plurality of substantially losslessly compressed medical images and a second storage area (20) which records a plurality of lossy compressed medical images corresponding to said losslessly compressed medical images.

## Description

This invention relates in general to a medical image storage system and more particularly relates to a transportable medical image storage medium, including a recordable compact optical disk (generally referred to as CD-R) having recorded thereon a plurality of losslessly compressed and corresponding lossy compressed medical images.

In traditional film screen radiography, an x-ray image of an object, such as an anatomical part of a patient, is formed in film. The film is then processed to produce a developed x-ray film image. Storage of and/or display of multiple x-ray film images from a patient study is space intensive, inconvenient and expensive. With the advent of digital image diagnostic scanner equipment, such as CT and MRI scanners, digital radiographic images have become commonplace. Digital images have several advantages over film images. They can be stored in compact magnetic or optical media. They can be displayed on a display device, such as a video monitor. They can also be processed to optimize various characteristics of the digital radiographic image.

Digital radiographic images can also be produced by means of computed radiography techniques. The following U.S. patents disclose the storage in an optical disk media of digital images produced by computed radiography: U.S. Patent 4,768,099, issued August 30, 1988, inventor Mukai; U.S. Patent 5,001,569, issued March 19, 1991, inventor Shigyo; U.S. Patent 5,019,975, issued May 28, 1991, inventor Mukai. It is disclosed in these patents that the image signals recorded on the optical disk are subjected to an image compression technique before recording. There is no disclosure in these patents, however, of recording both lossy and lossless radiographic images on the same optical disk. U.S. Patent 5,015,854, issued May 14, 1991, inventors Shigyo and others., discloses a radiation image display apparatus having a magnetic hard drive which stores both uncompressed image signals and a condensed image signal which represents an outline of the uncompressed radiation image. There is no disclosure in this patent, however, of storing the uncompressed and compressed radiation image signals on a recordable optical compact disk.

The most common method today to record cardiac catheterization images are in analog form on specialized 35 mm black and white cine film. However, there is a strong desire to archive the digitally generated images on a cost effective medium as the official record of the procedure and for future review as well. To use digital storage media more efficiently, the data can be mathematically compressed in a way that is totally reversible (lossless compression). To subsequently view the images, the clinician needs to see the images in real time motion and at slower speeds as well. The clinician also wants to randomly access rather than just sequentially access the images. Presently, there is no digital archive medium which can meet the following requirements: data transfer rates fast enough to provide real time motion display of losslessly compressed images and random data access. Therefore, the only available options to provide random access and real time motion display are, either to transfer the data from a slower inexpensive archive medium onto a faster more expensive buffer medium, or to have stored the data at a higher lossy compression so that the data transfer rate of the inexpensive archive medium is fast enough to support real time motion display.

Clinicians have expressed a strong preference to avoid the required delay time while the digital data is transferred from the archive medium to the fast buffer medium. Eigen (Nevada City, CA) introduced a product which stores the digital cardiac image data on a magneto-optical disk in two forms. One form is a lossless compression, the other is a lossy compression which supports higher speed motion display. The Eigen review product allows the clinician to review images in motion utilizing the lossy compressed data and if the user stops for a still image the lossless compression data corresponding to the still image will be utilized.

Using a magneto-optical disk to archive the images as a replacement for cine film has two distinct disadvantages, however. Firstly, cine film is an unalterable record of the procedure, whereas a magneto-optical disk is a re-writable medium permitting intentional or accidental changes to the permanent record of the procedure. Secondly, magneto-optical media violates the desire for a cost effective replacement for cine film. To archive an average procedure on a magneto-optical disk will cost about three times more than archiving the images on cine film.

According to the present invention, there is provided a solution to the problems of the prior art. According to an aspect of the present invention, medical image data is archived on a recordable optical compact disk. The medical image data is stored on the compact disk in two forms: losslessly compressed image data and corresponding lossy compressed image data.

According to another feature of the present invention, there is provided a transportable medical image storage medium comprising: a recordable optical compact disk; a first storage area of said recordable optical compact disk upon which are recorded a plurality of losslessly compressed digital medical images; and a second storage area of said recordable optical compact disk upon which are recorded a plurality of lossy compressed digital medical images corresponding to said losslessly compressed medical images.

The present invention has the following advantages:
a) The data is recorded on CD in at least two forms; one permits real time viewing of the images (lossy compression) while the other is the data preserved as the original (lossless compression). Either of these data forms are accessible.
b) Once the data are recorded onto the CD, that data cannot be either accidentally or intentionally modified since it is not a rewritable medium like magneto-optical disc is.
c) Since the CD can be recorded with ISO 9660 logical file structures, the CD files can be recognized by almost any common personal computer platform. This is unlike the magneto-optical disc, which does not have similar standards.
d) The recordable CD (CD-R) medium is presently equal to or less than the cost of cine film.
e) Using CD format optical medium provides a record with one patient record per CD, similar to today where there is only one patient's images on cine film. A single patient's record could be stored on large (physical) format media, however this would not be cost effective.
f) The physical nature of the CD-R medium and the format in which the data is encoded and written to the CD provides more robust storage of the archived data than other forms of optical media (including magneto-optical). This makes it possible to retrieve data from a CD which has scratches, dirt, and so forth and not retrieve data from other optical storage media with comparable physical abuse.

Fig. 1 is a block diagram of a medical image archiving and review system;
Fig. 2 is a diagrammatic view of an embodiment of record once compact disk;
Fig. 3 is a block diagram of an archiving station of the system of Fig. 1; and
Fig. 4 is a block diagram of a review station of the system of Fig. 1.

According to the present invention, there is provided a technique for recording medical images, and, more particularly, x-ray cardiology angiography images on a recordable optical compact disk. The recorded image data is compressed in two different ways: one is a lossy or irreversible compression and the other is a lossless or reversible compression. The lossy compression results in lost data. However, it should appear on a video display as nearly visually lossless. The lossless compressed digital data is compressed much less than the lossy compression and allows restoration of the image to its full original acquisitioned resolution. Lossless compression ratios are generally in the range of 1.5:1 to 4.0:1, whereas lossy compression ratios can be much higher, for example, 6:1 and up.

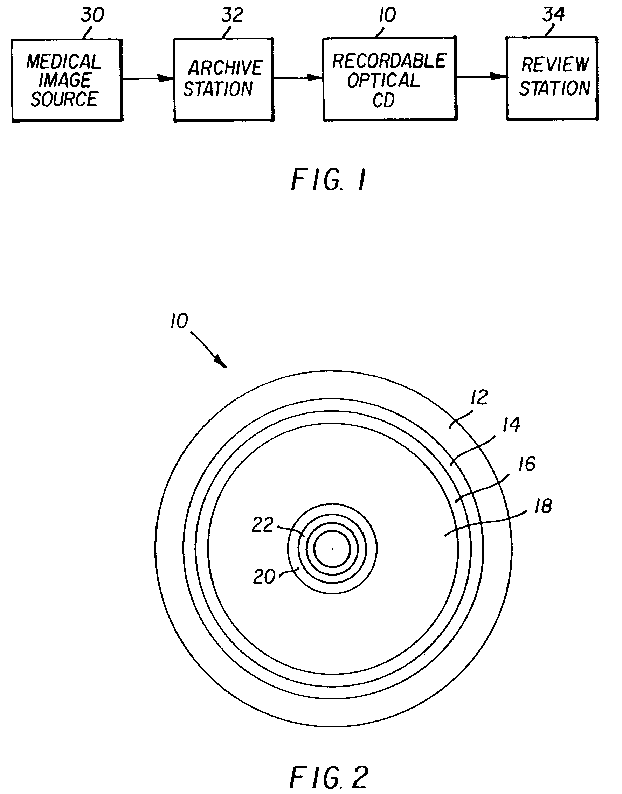

As shown in Fig. 2, the recordable optical compact disk 10 is formatted to have the following regions: a handling zone 12 (which is empty); a leadout region 14; a region 16 for storing the file directory; a region 18 where losslessly compressed digital image data is stored; a region 20 where the lossy compressed digital image data is stored; and a region 22 where header information is stored. Preferably, the header information contains the appropriate information to comply with ISO 9660 formats. The ISO 9660 compliance will enable multiple computer platforms (IBM compatible PC's, Macintosh, Sun, and so forth) to recognize files on the CD.

The recordable optical compact disk or CD-R is a compact disk medium that can be recorded on once, but which, once recorded on, is not erasable. The compact disk may be recorded on in unrecorded regions at different times. The medium is compact, transportable, cost effective, and reliable.

The recordable optical compact disk 10 of Fig. 2 can be used in a digital archiving and review system alternative to 35mm cine film and associated equipment (cine cameras, processors and projectors). Although a cardiology imaging system will be described hereinafter, it will be understood that other medical imaging archiving and review system applications are contemplated for use with the recordable optical compact disk of the present invention. As shown in Fig. 1, a cardiology motion image source 30 provides digital images to an archive station 32 where the images are recorded on one or more recordable optical compact disks 10. The recordable optical CD 10 can then be viewed on a review station 34.

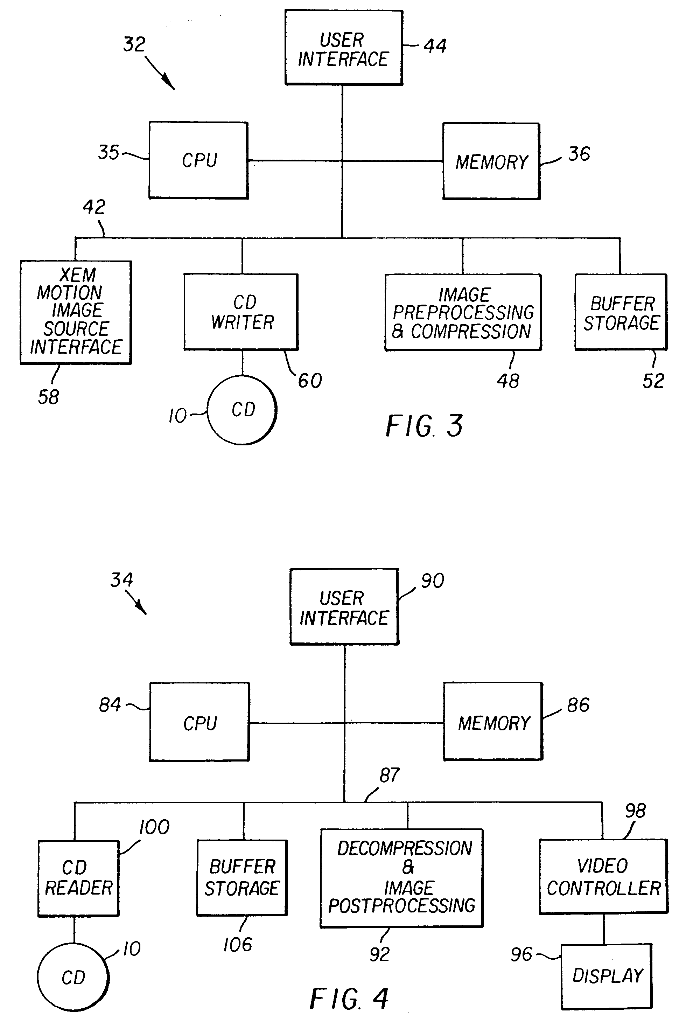

Referring now to Fig. 3, there is shown a block diagram of an archive station 32 for receiving cardiology images and recording them on a recordable optical compact disk 10. As shown, archive station 32 includes, a central processing unit (CPU) 35, internal memory 36, and a user input device linked to a bus 42. Bus 42 is linked to image pre-processing and compression board 48, and a buffer storage 52. Connected to bus 42 is an XEM (X-ray equipment manufacturer) motion image source interface 58, and a CD writer 60. CD writer 60 is a recordable optical disk writer, such as supplied by the Eastman Kodak Company, Rochester, New York, as the Kodak PCD Writer 600. The compression board is preferably based on the Joint Photographic Experts Group (JPEG) international digital image compression standard for continuous tone still images. A general overview of this standard is presented in the article entitled "The JPEG Still Picture Compression Standard", by Gregory K. Wallace, Communication of the ACM, April 1991, volume 4, No. 4, pages 30-44. It will be understood that other compression techniques can also be used.

The operation of archive station 32 is as follows:

In general, during the cardiac catheterization case study, the images are stored in the x-ray equipment manufacturer's (XEM's) digital acquisition memory. The entire case's data will be transferred to the archive station 32. Once data is stored in station 32, the XEM memory is free to acquire a new patient's images. The image data is received from the XEM memory by interface board 58. The image data is compressed two different ways in compression board 48: there is a lossy compression and a lossless (for example, 2:1) compression. The lossy compression results in lost data. However, it should appear as nearly visually lossless upon display. The 2:1 compression is lossless and allows restoration of the image to its full original acquisition resolution. The lossless and lossy compressed image data is stored in buffer storage 52. The user enters the patient's name, ID number, and so forth into the archive station 32 by user input device 44, if this information cannot be supplied via the XEM motion image source interface 58. This information is merged with the image data and written by CD writer 60 to the CD 10. This feature allows the elimination of preparing the lead letters and the identification x-ray exposure. Part of this information will be included in the header information written to the CD 10 as well as the file directory of the CD 10 contents.

A typical cardiology motion image source produces 30 digital images per second. Each digital image can have a format of 512 x 512 pixel array, or a 1024 x 512 pixel array, with a pixel depth of 8 bits. If a typical patient case study includes 2000 images, a single Recordable CD can be used to record the case study (assuming lossy compression of 6:1 and lossless compression of 2:1).

Because of the probable need to accept new data from the user's lab while the previous patient's data has not been completely archived, buffer storage 52 has the capacity to hold a minimum of two long patient study cases.

Currently available 63 minute record once CDs 10 have a maximum capacity of 580 Megabytes. The total capacity is not just data, but must include the data plus the ISO 9660 file structures, and so forth Greater capacity record once CDs should be physically interchangeable with current record once CDs.

If the cath case exceeds the capacity of a single CD 10, the lossy compressed track will contain the images of the entire case and is identical on each CD 10. The lossless data is appropriately split between the multiple CDs 10.

A review station 34 for displaying the images of a medical image study recorded on a recordable CD 10 is snown in Fig. 4. As shown, review station 34 includes a user interface 90, a central processing unit (CPU) 84, and memory 86 connected to bus 87. Also connected to bus 87 is an image decompression and post-processing board 92, and video display 96 by way of video controller 98. Review station 34 includes, a CD reader 100, and buffer storage 106 connected to bus 87.

In general, review station 34 operates as follows: A CD 10 is loaded into CD reader 100. Reader 100 produces sequential images of the medical image study. The images are stored in buffer storage 106. Images which are read out of buffer storage 106 are decompressed and processed by board 92. The images are displayed on display 96 by way of video controller 98.

Once the medical image case is stored onto CD 10 by archive station 32, it is available for immediate review on review station 34 (Fig. 4). The user selects through user interface 90 the desired CD 10 for viewing and the desired review mode. The reader 100 reads the CDs 10 data compression format to determine the appropriate image decompression factors. The file directory is read from the CD 10 into memory 86 and the review station 34 is ready to begin displaying images on display 96.

The need for palindrome capability (viewing motion both forward and reverse) has led to two operational modes for review station 34; one of which can offer full palindrome viewing if the system is restricted to utilizing the lossy data. Furthermore, the user only has to load one of the potentially multiple CDs 10 used to archive the case, since each CD contains identical lossy compression data of the entire case. The other mode will not provide reverse viewing, but will allow the user to back up and repeat viewing forward motion while maintaining the ability to access the lossless data for slow speed or still viewing. This mode of operation will require the user to load all CDs 10 involved in archiving the case.

Although the higher compression image data has lost data from the original image data, its compression will produce nearly visually lossless images. Cardiologists have consistently expressed a reluctance to wait for digital image records to be transferred and loaded into memory for real-time viewing. This concern is obviated by having the lossy compressed images available for real-time viewing (and also slow/still speeds depending upon the mode of operation) while the lossless images are available for detailed study of the images at slow or still speeds. The user can switch rapidly between any of the speeds: real-time, slow or still images.

### MODE A

If the user selects Mode A, only the lossy data will be utilized for any speed between still and real time motion. As the lossy compressed data is read from the CD 10 it is loaded into the buffer storage 106 which will act as a buffer to compensate for the variable amount of compressed data on a frame to frame basis. After a suitable amount of data has been buffered that will allow sustained display rates, the data is sent to the decompression board 92 for image display on display 96. When the user switches to reverse viewing, the compressed data will be read in "reverse" from buffer storage 106 and sent to the decompression board 92 for processing. Via either data direction path, the decompressed data is then processed for display 96.

### MODE B

If the user selects Mode B, both the lossy and lossless data will be utilized for image display on display 96. Obviously, if the patient case was archived with multiple CDs 10, potentially the user must load all the cases CDs 10. The lossy data will be used for viewing images in forward motion at speeds > the threshold speed through realtime motion. For images viewed still, single advance or less than the threshold speed, the lossless data will be obtained from the CD 10 for decompression by decompression board 92. The threshold speed is a function of the data transfer rate of the CD reader 100 used and the amount of data per image. The amount of data per image is primarily a function of the data acquisition/archive matrix.

Since the user can interactively switch between the lossy and lossless data, the scheme of loading the compressed data into buffer storage 106 and reading it in reverse will not work as the reverse viewing of the lossy/lossless data image mixture would not permit sustained viewing speeds and this would be very annoying to the user. Therefore, true palindrome viewing cannot be provided when operating in this mode. To repeat viewing a sequence of images, the user may "jump back" a specific number of images and once again view images in forward motion. Depending upon frames to be viewed and the speed selected for reverse viewing of images, the user may experience a time delay as the appropriate data (lossy or lossless) may have to be first read off the CD 10. In Mode B, motion is limited to the forward direction only.

## Claims

1. A transportable medical image storage medium comprising:
a recordable optical compact disk (10);
a first storage area (18) of the recordable optical compact disk (10) which records a plurality of substantially losslessly compressed digital medical images; and
a second storage area (20) of the record once optical compact disk which records a plurality of lossy compressed medical images corresponding to the losslessly compressed digital medical images.

2. The medium of claim 1 wherein the first storage area (18) is separate from the second storage area (20) on the compact disk (10).

3. The medium of claim 1 including a third storage area (16) on the compact disk (10) which incudes a file directory of images recorded in the first and second storage areas of the compact disk (10).

4. The medium of claim 1 including a fourth storage area (22) on the compact disk (10) which includes header data, such as, logical file structures.

5. The medium of claim 4 wherein the header data (22) conforms to the ISO(International Standards Organization) 9660 format.

6. The medium of claim 1 wherein the digital medical images recorded on the medium are cardiology images.

7. The medium of claim 1 wherein the digital medical images recorded on the medium are a sequence of motion cardiology images.

8. The medium of claim 7 wherein the sequence of motion cardiology images recorded on the medium are from a single patient case study.

9. The medium of claim 1 wherein the substantially losslessly compressed digital medical images and the lossy compressed digital medical images recorded on the medium are compressed in general conformity with the JPEG(Joint Photographic Experts Group) Still Picture Compression Standard.
